# EUROPEAN PATENT SPECIFICATION

(11) **EP 0 345 317 B1**
(45) Date of publication and mention of the grant of the patent: **25.08.1993**
(21) Application number: 88910379.2
(22) Date of filing: 08.11.1988
(51) Int. Cl.: A61M 31/00

(54) **MICROCRYSTALLINE MATRIX FOR ADMINISTRATION OF BENEFICIAL AGENT**
MIKROKRISTALLINE MATRITZE ZUR VERABREICHUNG EINES HEILMITTELS
MATRICE MICROCRISTALLINE D'ADMINISTRATION D'AGENTS THERAPEUTIQUES

(30) Priority: 16.11.1987 US 121316
(43) Date of publication of application: 13.12.1989
(73) Proprietor: BAXTER INTERNATIONAL INC. (a Delaware corporation), Deerfield Illinois 60015 (US)
(72) Inventor: WEINLESS, Naomi, L., Highland Park, IL 60035 (US); POKROPINSKI, Sharon, Berwyn, IL 60402 (US)
(74) Representative: MacGregor, Gordon
(86) International application number: US8803983
(87) International publication number: WO8904687

(56) References cited:
- EP-A- 0 059 694
- EP-A- 0 192 367
- CH-A- 497 181
- GB-A- 1 476 057
- GB-A- 2 054 599
- US-A- 25 129
- US-A- 2 987 445
- US-A- 4 552 555
- US-A- 4 738 668
- US-A- 4 758 430
- Transfusion, Volume 24, no. 6, issued 1974, (Philadelphia), B.A. Myhre et al., "Preservation of red cell antigens during storage of blood with different anticoagulents", see pages 499-501

## Description

The invention relates to a method of controlled administration of a beneficial agent to blood or a parenteral fluid or peritoneal dialysis fluid passing through a conduit, according to the first part of claim 1. Such a method is known from EP-A-0 059 694. Furthermore, it relates to a beneficial agent administration device according to claim 8 and to a use of citrate or mannitol in a tablet, according to claim 9

In a United States patent application entitled Controlled Administration of Beneficial Agent to Blood, and in another United States patent application entitled Glassy Sugar Matrix for Controlled Administration of Beneficial Agent, the use of dry materials containing beneficial agents is disclosed as a vehicle for conveying such beneficial agents to a medical solution such as blood, or simpler parenteral solutions such as saline solution, glucose solution, or the like. Preferably, a solid mass, and typically a tablet, of beneficial agent is positioned typically in an enlarged chamber in an administration, blood collection, or processing set, so that as the medical solution passes through the administration set, a controlled amount of the beneficial agent dissolves into the passing medical solution in a manner that is substantially uniform or otherwise predetermined. Generally, the beneficial agent comprises a sugar or sugar alcohol in a substantially anhydrous glass form, typically with other beneficial agents admixed with the sugar. Such beneficial agents may be citrate in the form of citric acid and sodium citrate, and possibly including a sodium phosphate salt, to provide an anticoagulant for blood which may be uniformly administered to various aliquots of blood passing through the system, particularly the early aliquots of the flow which in the present technology may be exposed to an excessive concentration of anticoagulant, with undesirable effects on the living cells in those early flow portions of blood. In the US patent applications cited above, the beneficial agent in substantially anhydrous, glass form is capable of assuming the glassy form (of the order of hard candy), because a significant percentage of the formulation is a sugar or sugar alcohol.

In accordance with this invention, a method is provied of controlled administration of a beneficial agent to blood or a parenteral fluid or peritoneal dialysis fluid passing through a conduit, wherein the blood or fluid is passed over a solid mass comprising the beneficial agent and a carrier for the beneficial agent, characterised in that the carrier is citrate or mannitol in microcrystalline, substantially anhydrous form. The solid mass may be substantially free of sugar, or the sugar may be present in a relatively low concentration. Such a solid mass provides significant benefit when used in conjunction with diabetic patients, for example, permitting administration of beneficial agents from a dry form. Additionally, the carrier of this invention can provide superior protective action to a beneficial agent carried within it against moisture, oxygen, and even gamma radiation, to reduce the loss of a beneficial agent which may be sensitive to any of the above.

### DESCRIPTION OF THE INVENTION

In this invention, a method is provided for passing medical solution, typically blood but also including any desired parenteral solution, or the like, through a conduit, typically into a container. In accordance with this invention, one passes the medical solution across a solid mass comprising a carrier and the beneficial agent. The carrier comprises a citrate or mannitol in microcrystalline, substantially anhydrous form.

Preferably, at least 70 weight percent of the solid mass is citrate (i.e. citrate salts and/or citric acid) or mannitol, there being preferably no more than 20 weight percent of sugar present. The solid mass is shaped and proportioned whereby a controlled amount of the beneficial agent dissolves into passing medical solution in a manner that is substantially uniform or otherwise predetermined. Alternatively, nonuniform administration rates may be predetermined, depending on the shape and condition of the solid mass.

In one typical use of the invention of this application, the medical solution may be blood, for example blood which is in the process of being collected from a donor and passed into the donor bag of a single or multiple blood bag system which may be of conventional design and use, but for the modifications provided by this invention. In this circumstance, the solid mass may comprise citrate as a carrier for any known anticoagulant or other blood cell preservative, such as platelet preservatives. Additionally, a water soluble, non-toxic source of phosphate may be present in a concentration effective to promote blood storage in known manner. Adenine may be present, if desired, as well as any other ingredient which is desired for the storage of blood or components thereof.

By the term "blood", it is intended to include not only whole blood, but components of blood as may be desired, for example blood plasma (either platelet rich or platelet poor) or suspensions of packed red cells, white cells, or platelets in saline solution, any other appropriate suspension of blood cells, or the like.

In addition to the beneficial agent and carrier, typical sugars or sugar alcohols may be present in minor proportions. They should be soluble, non-toxic sugars or sugar alcohols which may or may not be metabolizable as a nutrient according to the desires of the formulator and the use to which the mass of beneficial agent is to be put.

Examples of usable citrates which may be used in the manner described above include sodium citrate, and citric acid. The proportions of sodium citrate and citric acid may, for example, be adjusted to obtain the pH desired.

For example, a mixture of 5.6 molar parts of sodium citrate to one molar part of citric acid, intimately mixed and formed into a microcrystalline tablet will dissolve to yield in distilled water a solution having a pH of 5. A mixture of 133.3 molar parts of sodium citrate per one molar part of citric acid will dissolve to yield in distilled water a pH of 7.4. Thus, it can be seen that a desired pH can be obtained from the dry material used in this invention by control of the citrate to citric acid ratio. This ratio may typically range from 2 to 200/1, and is more preferably 5 to 150/1, expressed in molar parts.

The dry, microcrystalline, solid mass is preferably provided in the form of one or more dry tablets, with means provided in the conduit for carrying the solid mass and permitting blood or other medical solution flowing through the conduit to enter into contact with the solid mass. As solution passes through the solid mass, it picks up a substantially predetermined amount of beneficial agent and carries it away. The geometry of the preferred tablet may be specifically shaped to provide a desired, controlled, relatively constant release of the beneficial agent as the solution passes across it. Specifically, a single tablet may be provided which is relatively long and wide relative to its thickness. With such a configuration, as the tablet erodes, there is little change in the surface area of the tablet, so that the amount of beneficial agent which dissolves into passing medical solution remains substantially uniform, at least until the tablet begins to disintegrate. It may be desirable to have the tablet or tablets of a desired size and thickness so that the last of the medical solution has passed through the conduit before the tablet or tablets erode away so much that they begin to break up, at which point a significant difference in the transfer rate of beneficial agent into the solution can take place. Otherwise, the complete tablet or tablets may be dissolved in the process, giving visual indication that the complete, desired dose has been administered.

The dry solid mass may be a molded, extruded, or pressed structure, which may be flat and thin as described above, or the tablet may be a pressed, solid structure having multiple flow channels through it for flow of medical solution therethrough, or of a cylinder shape so that medical solution flows through its bore, or any other shape or structure as desired. By this invention, since the geometry and size of the solid mass can be controlled, improvements in control of transfer of the beneficial agent to the medical solution are obtained. For example, an increased amount of beneficial agent may be initially provided by a tablet having a porous outer portion and a denser, less porous inner portion.

Thus, the use of the substantially dry solid mass typically positioned in a conduit, provides significant advantages. The empty bag or the receptacle which thus may be used for blood or other medical solution is sterilized with much greater ease than current blood bags which contain liquid anticoagulant. Additionally, transportation and handling advantages are achieved, when compared particularly with containers of frozen drug solution, which must be transported in frozen condition. The drug is simply incorporated into the citrate or mannitol carrier. If desired, a parenteral solution such as saline may be passed over the mass and directly into the patient, carrying the dissolved citrate and a controlled amount of drug with it. Thus, critical drugs may be administered to a patient without a special step of reformulation of the dry drug into a parenteral solution.

Therefore, significant advantages of manufacturing, transportation, storage, and handling are provided.

Additionally, citrate and mannitol microcrystalline carriers having a low water content (for example 2 to 5 percent by weight) may be good oxygen barriers. Thus, oxygen-sensitive drugs may be protected while incorporated in the carrier used in this invention.

Also, the solid mass of this invention may be well suited for gamma irradiation sterilization, and moisture sensitive beneficial agents are also protected by storage in the carrier in accordance with this invention. It is believed that the citrate or mannitol present can exert a protective action on beneficial agents which are mixed with the citrate or mannitol prior to contact with the medical solution for use, providing protection against moisture, oxygen, and even gamma radiation, to reduce loss of effective agent during gamma irradiation sterilization, for example.

Without wishing to be limited to any theory of operation of the invention, it is believed that the hydroxyl groups of particularly mannitol present can act as free-radical scavengers to protect sensitive, beneficial agents present from degradation during or following gamma irradiation for sterilization.

Additionally, the solid masses used in this invention are resistant to bacterial attack due to their low water content. It is generally preferred for the water content of such solid masses to be about 1 to 2 percent, and typically no more than about 5 percent by weight.

Additionally, dry solid masses comprising a citrate carrier as in this invention can be effectively molded during manufacture to form a tablet or tablets of desired dimensions to obtain the exact, desired dissolution rate as medical solution passes in contact therewith.

Typically at least 30 weight percent of mannitol or citrate salt and citric acid are present in the formulation as a vehicle for delivery of a beneficial agent.

Examples of beneficial agents that may be included in the citrate or mannitol carrier of this invention include drugs that are desirably released on a controlled release basis, for example, aspirin, antibiotics, thyroid hormones, cancer chemotherapy agents, or insulin. Accordingly, the uniformity of release which is available through one or more tablets of the solid mass of this invention may facilitate the controlled administration of such drugs.

As another use, the solid mass of this invention may be reconstituted with peritoneal dialysis solution, for example CAPD solution, to place medicaments and nutrients such as ketoacids into the CAPD solution. Likewise, collected blood may be brought into contact with a microcrystalline citrate carrier containing an anticoagulant for short term anti-coagulation of blood, for example, for use in a plasmapheresis or other procedure involving the short term extracorporeal storage of blood. For example, this technique may prove advantageous for use with diabetics by avoiding the use of an anticoagulant having larger quantities of sugar.

### DESCRIPTION OF DRAWINGS

Fig. 1 is a plan view of a blood collecting set made in accordance with this invention, shown to be integrally connected to a medical solution storage bag; and
Fig. 2 is a sectional view taken along line 2-2 of Fig. 1.

### DESCRIPTION OF SPECIFIC EMBODIMENTS

Referring to the drawings, there is shown a set 10 for collecting blood from a patient and conveying it to blood storage container 12, which may be made in substantially conventional manner. Set 10 carries at one end a conventional blood connection needle 14 for collecting blood from the venous system of the patient, which blood then passes through set 10 into blood bag 12.

The conduit of set 10 defines an enlarged portion 16 which includes a microcrystalline, solid mass 18 typically having a water content on the order of no more than about 2-5 percent by weight. Tablet 18 is shown to be made in the form of thin, rectangular member positioned within enlarged portion 16. The inner walls of both sides of enlarged portion 16 define an array of small projections 20 which project inwardly to define flow passages between the inner wall of enlarged portion 16 and the surface of tablet 18. Alternatively, separate flow screening may replace the projections 20 as flow passage defining means.

Specifically, tablet 18 may include, for example, a dried mixture of:

| | |
|---|---|
| Sodium Citrate | 50 g(gm). |
| Citric Acid | 10 g(gm). |

The above ingredients are dissolved as a near saturated solution in water by heating to boiling at about 115°C (239 degrees F.). Then about 1 per cent by weight of dry sodium citrate powder is mixed into the solution, in order to promote crystal growth. The mixture is then spooned into molds and placed in a heated oven at 1.01x10⁵ N/m² (30'') of vacuum for about 12 hours, until the desired water content of about 2-5 per cent or less by weight is reached (the water content being as defined by its temperature at the air pressure obtained by the vacuum system). The viscous mass is then cooled to obtain a microcrystalline mass. The term "microcrystalline" means that the crystals present are predominantly less than 50µm (microns) in size.

The beneficial agent, for example an anticoagulant, may be added when the temperature of the molten citrate falls to about 93.33°C (200 degrees F). or below, with vigorous stirring or alternatively in a twin screw extruder, to form a homogeneous mass. This material is molded into the desired shape and allowed to solidify, to form the microcrystalline solid mass in the shape of tablet 18, which may be then placed in portion 16.

Blood bag 12 may be initially free of any anticoagulant. The entire system, including tablet 18, is typically radiation sterilizable, since it is substantially free of moisture. Additionally, no overpouch or other outer package is required to prevent the loss of water from a liquid anticoagulant present, which constitutes a significant advantage over the conventional, present blood collection equipment.

For use, a conventional phlebotomy is made into a vein of a blood donor with needle 14, and the blood flows through conduit 10. As it flows, it enters enlarged portion 16, where it flows in a pair of opposed flow halves 22 between the walls 24 of enlarged portion 16 and the flat surfaces of tablet 18. As the blood so flows, tablet 18 is dissolved away into the flowing blood at typically a relatively uniform rate of dissolution, so that each individual portion of the flowing blood passing through conduit 10 picks up a substantially similar amount of the dissolved beneficial agent of tablet 18 as it flows across it. From there, the flowing blood, carrying the dissolved beneficial agent, e.g., anticoagulant, passes into bag 12 for storage, without encountering an excessive concentration of anticoagulant or other beneficial agent.

Any design of blood bag or other container may also be utilized in conjunction with this invention. If desired, the tablet 18 may be encapsulated and released via diffusion through a semipermeable membrane.

Additionally, the invention of this application may be used in conjunction with various apheresis procedures, for example bag apheresis hardware collection, apheresis, or continuous apheresis procedures. For example, medications may be administered to blood in accordance with this invention by an apheresis procedure or the like.

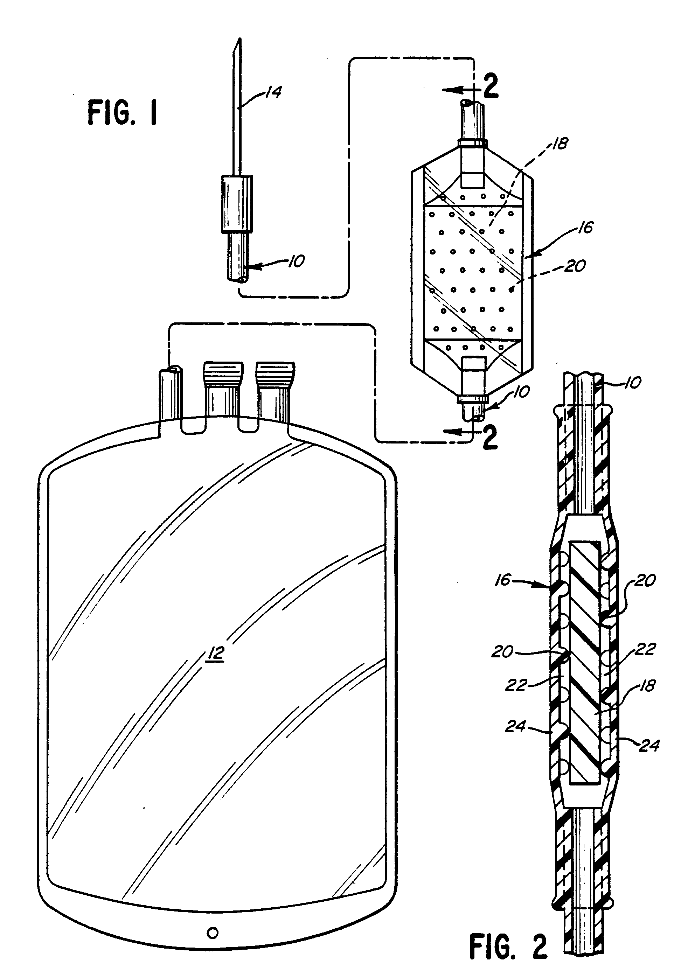

An antibiotic or other drug may be mixed into the molten citrate when it is at a temperature of about 93.33°C (200 degrees F). as described above. After intimate mixing, the molten mixture is molded into tablet form, or any other desired shape, and allowed to solidify to a solid form. The resulting tablets may be placed into a chamber similar to enlarged portion 16 of set 10 which may be adapted for connection, or integrally connected with, a bag of parenteral solution. Fig. 1 in this circumstance can represent such a system, in which bag 12 contains, for example, sterile normal saline solution, or even pure water, although such a system might not be fail safe since intravenously administered water is very damaging to blood cells.

In this instance, connection with the venous system of a patient is made by needle 14, and the parenteral solution in bag 12 is allowed to pass from bag 12 through set 10 into the patient's venous system. As it passes through enlarged portion 16, the citrate and admixed beneficial agent of tablet 18 dissolves into the passing parenteral solution at a predetermined rate, so that the desired amount of drug may be administered simply by administering a desired quantity of parenteral solution from bag 12 at a predetermined rate of flow.

Thus, the invention of this application may not only be used for the handling of blood, but it may be used for the administration of beneficial agents directly to the patient, or to another container, in which solution from a bag is passed through a conduit which contains the desired beneficial agent. Such a system is very flexible as to the amount of beneficial agent which is delivered, contrary to many present systems. If one wishes to deliver only one third of the available beneficial agent in tablet 18, one can simply administer a predetermined amount of solution from bag 12 through set 10 to the patient, and then terminating such administration when the desired amount of beneficial agent has been delivered. Additionally, the enlarged chamber 16 may be manufactured in a form of a cartridge for connection to parenteral solution sets, with the various cartridges carrying different types of beneficial agents as may be desired in the mass of citrate or mannitol. The doctor then needs to only select a cartridge which carries the beneficial agent of the desired type and concentration, and administer it to the patient without a special drug reconstitution step previous to the administration.

Examples of beneficial agents which may be inserted into the citrate or mannitol carrier of this invention include anticlotting agents such as heparin, antibiotics of any type such as Gentamycin, penicillin, or erythromycin, strongly biologically active drugs that require controlled administration to the patient such as thyroid hormones or insulin, and the like.

The following is a specific example of how a tablet 18 including a mannitol carrier may be utilized for delivery of the mannitol and the beneficial agent that it contains to a patient.

Seventy-five grams of mannitol are dissolved in 125 ml. of sterile water which is boiling at 100 degrees C. After the mannitol has been mixed into the water, it is heated to 115 degrees C., as permitted by the boiling point elevation provided by the mannitol.

The solution is then removed from heat, and 2 grams of powdered mannitol are added to promote microcrystallization. After the crystallization process is completed in a matter of five minutes or so, one spoons the viscous mass into tablet molds to form desired tablets, for example of the design of tablet 18.

The filled tablet molds are evacuated at 7.74x10⁴ N/m² (23 inches of mercury) for one hour while cooling proceeds. Following this, the evacuated chamber is heated to 55 degrees C. for one hour to facilitate further water removal. The tablet molds are allowed to remain under evacuated conditions overnight.

Following this, the molds are opened to obtain the mannitol tablets. These tablets may be used in the manner of tablet 18 in the apparatus described above to provide a predetermined dose of beneficial agent to parenteral solution flowing across tablet 18.

Accordingly, by the invention of this application, a system is provided in which a beneficial agent can be reconstituted into its beneficial form at the site of use without intermediate diluent, if desired. The beneficial agent of this invention can be directly reconstituted into blood, where its beneficial action takes place directly on the blood without the need for an intermediate diluent.

## Claims

1. A method of controlled administration of a beneficial agent to blood or a parenteral fluid or peritoneal dialysis fluid passing through a conduit, wherein the blood or fluid is passed over a solid mass comprising the beneficial agent and a carrier for the beneficial agent, characterised in that the carrier is citrate or mannitol, in microcrystalline substantially anhydrous form.

2. The method of Claim 1 in which at least 30 weight percent of solid mass is citrate or mannitol.

3. The method of Claim 1 in which said solid mass contains no more than 20 weight percent of sugar.

4. The method of Claim 1 in which said solid mass is substantially free from sugar.

5. The method of any preceding claim in which said solid mass is shaped and proportioned whereby a controlled amount of said beneficial agent dissolves into passing solution in a manner that is substantially uniform.

6. The method of any preceding claim in which said solid mass contains citrate in the form of a mixture of an alkali metal citrate and citric acid.

7. The method of Claim 6 in which said alkali metal citrate is sodium citrate.

8. A beneficial agent administration device, for use in the method of Claim 1, the device comprising a conduit having a portion (16) in which is contained a tablet formed as a solid mass comprising a beneficial agent and a carrier therefor, the carrier being citrate or mannitol in microcrystalline, substantially anhydrous form.

9. Use of citrate or mannitol in microcrystalline, substantially anhydrous form as a carrier for a beneficial agent in the manufacture of a tablet for the purpose of reducing the loss of beneficial agent exposed to moisture, oxygen or gamma radiation.

## Patentansprüche

1. Verfahren zur kontrollierten Verabreichung eines gesundheitsfördernden Mittels zu Blut oder einem parenteralen Fluid oder einem Peritonealdialysefluid, das durch eine Leitung strömt, wobei das Blut oder Fluid über eine feste Masse geleitet wird, die das gesundheitsfördernde Mittel und einen Träger für das gesundheitsfördernde Mittel aufweist, dadurch gekennzeichnet, daß der Träger Citrat oder Mannitol in mikrokristalliner, im wesentlichen wasserfreier Form ist.

2. Verfahren nach Anspruch 1, wobei wenigstens 30 Gew.-% der festen Masse Citrat oder Mannitol sind.

3. Verfahren nach Anspruch 1, wobei die feste Masse nicht mehr als 20 Gew.-% Zucker enthält.

4. Verfahren nach Anspruch 1, wobei die feste Masse im wesentlichen zuckerfrei ist.

5. Verfahren nach einem der vorhergehenden Ansprüche, wobei die feste Masse geformt und abgemessen wird, wodurch eine kontrollierte Menge des gesundheitsfördernden Mittels sich auf eine im wesentlichen gleichmäßige Weise in vorbeiströmende Lösung löst.

6. Verfahren nach einem der vorhergehenden Ansprüche, wobei die feste Masse Citrat in Form eines Gemischs von einem Alkalimetallcitrat und Citronensäure enthält.

7. Verfahren nach Anspruch 6, wobei das Alkalimetallcitrat Natriumcitrat ist.

8. Vorrichtung zur Verabreichung eines gesundheitsfördernden Mittels zur Verwendung bei dem Verfahren nach Anspruch 1, wobei die Vorrichtung eine Leitung mit einem Bereich (16) aufweist, der eine Tablette enthält, die als eine feste Masse gebildet ist, die ein gesundheitsförderndes Mittel und einen Träger dafür aufweist, wobei der Träger Citrat oder Mannitol in mikrokristalliner, im wesentlichen wasserfreier Form ist.

9. Verwendung von Citrat oder Mannitol in mikrokristalliner, im wesentlichen wasserfreier Form als ein Träger für ein gesundheitsförderndes Mittel bei der Herstellung einer Tablette zu dem Zweck, den Verlust an gesundheitsförderndem Mittel, das Feuchtigkeit, Sauerstoff oder Gammastrahlung ausgesetzt ist, zu reduzieren.

## Revendications

1. Procédé d'administration contrôlée d'un agent thérapeutique dans le sang ou un fluide parentéral ou un fluide de dialyse péritonéale passant dans un conduit, dans lequel on fait passer le sang ou le fluide sur une masse solide contenant l'agent thérapeutique et une substance porteuse de l'agent thérapeutique, caractérisé en ce que la substance porteuse est un citrate ou du mannitol, sous forme microcristalline sensiblement anhydre.

2. Procédé suivant la revendication 1, dans lequel le citrate ou le mannitol représente au moins 30% en poids de la masse solide.

3. Procédé suivant la revendication 1, dans lequel ladite masse solide ne contient pas plus de 20% en poids de sucre.

4. Procédé suivant la revendication 1, dans lequel ladite masse solide est sensiblement exempte de sucre.

5. Procédé suivant l'une quelconque des revendications précédentes, dans lequel ladite masse solide est configurée et dimensionnée de sorte qu'une quantité réglée dudit agent thérapeutique se dissout dans la solution en circulation, d'une manière qui est sensiblement constante.

6. Procédé suivant l'une quelconque des revendications précédentes, dans lequel ladite masse solide contient un citrate sous la forme d'un mélange d'un citrate de métal alcalin et d'acide citrique.

7. Procédé suivant la revendication 6, dans lequel ledit citrate de métal alcalin est le citrate de sodium.

8. Dispositif d'administration d'agent thérapeutique, utilisable dans le procédé de la revendication 1, ce dispositif comprenant un conduit ayant une partie(16) dans laquelle est contenu un comprimé sous la forme d'une masse solide composée d'un agent thérapeutique et d'une substance porteuse pour cet agent, la substance porteuse étant un citrate ou du mannitol sous forme microcristalline sensiblement anhydre.

9. Utilisation de citrate ou de mannitol sous forme microcristalline sensiblement anhydre comme substance porteuse pour un agent thérapeutique, dans la fabrication d'un comprimé, dans le but de réduire la perte d'agent thérapeutique lors de l'exposition à l'humidité, à l'oxygène ou à un rayonnement gamma.
